# EUROPEAN PATENT APPLICATION

(11) **EP 4 483 832 A1**
(43) Date of publication of application: **01.01.2025**
(21) Application number: 23306060.7
(22) Date of filing: 29.06.2023
(51) Int. Cl.: A61B 34/30, A61B 1/00, A61B 90/50, A61B 17/00, A61B 90/00

(54) **SURGICAL SYSTEM FOR EAR, NOSE, THROAT SURGERIES**

(71) Applicant: Zentact Robotics, 38400 Saint Martin d'Hères (FR)
(72) Inventor: LOMBARD, Bertrand, 69100 VILLEURBANNE (FR)
(74) Representative: Regimbeau

(57) **Abstract**

An object of the present disclosure concerns a surgical system (100) comprising at least two robotic assemblies (110) and a holder (120), each robotic assembly (110) comprising at least one robotic arm (111) and one medical instrument (112), the robotic arm (111) presenting at least two motorized degrees of freedom and the medical instrument (112) being held by the robotic arm (111), wherein each medical instrument (112) is supported by the holder (120), and wherein a non-null distance is formed between two adjacent medical instruments (112) this non-null distance being measured at any point between the concerned robotic arm (111) and the holder (120).

## Description

The present disclosure relates to a surgical system adapted to be used to perform minimally invasive surgeries, and particularly to a surgical system particularly adapted to be used to perform minimally invasive surgeries on soft tissues, such as ENT (Ear, Nose, Throat) surgeries.

Currently, the ENT surgeons have to hold, manually, an endoscope which allows them to see inside the cavity where they have to perform the concerned surgery and one or several surgical instrument(s) to actually perform said surgery. Holding and actuating simultaneously as many objects renders their manipulation particularly complex and thus leads to long learning curves and multiple potential errors.

During the last few decades, several robotized, or partially robotized, surgical systems have been developed to improve accuracy and safety of minimally invasive surgeries. In particular, significant progress has been made for laparoscopic type systems, where the point of entry becomes the pivot point of the instrument used. One major drawback of these surgical systems is that they do not permit to manipulate two or more medical instruments in a small and confined area within the body, such as an ear canal. To work in such a small and confined area impose to manipulate medical instruments arranged very close to one another and extending almost parallel to one another, to stay within said anatomic canal boundaries.

For instance, document US20190374129A1 discloses an endoscope adapted to be inserted through small incision or natural orifices of a human body and equipped with tracking sensors, thus permitting to the operator to know the current pose of said endoscope within the body of the treated patient. Even if this kind of system improves the spatial coordination with respect to image capture, it requires a long learning process for the surgeon as he/she must coordinate his/her movements with the movements of the endoscope displayed on a display.

Document US20180214226A1 describes a robotic system which permits to drive, tele-operatively, at least two flexible instruments, both received in a canula extending through an incision realized in the skin of the patient. This kind of system also requires a long learning process for the surgeon as tele-operating flexible instruments manipulation is a complex task.

Document US20140195048A1 finally describes a robotic system particularly adapted to operate laparoscopies, with four robotic arms which respectively hold one surgical instrument. This robotic system is well adapted to perform said laparoscopies wherein several incisions can be realized in the patient's skin. Such kind of robotic however is not adapted to perform ENT (ear, nose, throat) surgeries which require to have at least two, often three, distinct instruments passing through a small natural channel, such as an ear canal for instance. The robotic system described in the document is indeed way too cumbersome to be used for this kind of surgeries.

Finally, all the systems disclosed above aim at stabilizing movements of the endoscope but fail to provide stability to the surgeon's hands holding the surgical instruments used to actually perform the concerned surgery.

Thus, there remains a need for an improved robotic system permitting to direct several medical instruments, while making them pass through one small natural canal.

The present disclosure falls within this context and aims at solving at least the presented drawbacks.

An object of the present disclosure thus concerns a surgical system comprising at least two robotic assemblies and a holder, each robotic assembly comprising at least one robotic arm and one medical instrument, the robotic arm presenting at least two motorized degrees of freedom and the medical instrument being held by the robotic arm, wherein each medical instrument is supported by the holder, and wherein a non-null distance is formed between two adjacent medical instruments this non-null distance being measured at any point between the concerned robotic arm and the holder.

The surgical system can be further complemented by one or several of the following features taken alone or in combination.

The surgical system comprises at least three robotic assemblies.

Each robotic arm comprises at least four motorized degrees of freedom.

Each medical instrument extends, at least between the robotic arm and the holder, along a respective main axis of extension, the holder being movable along at least one translational degree of freedom transversal to the main axis of extension of each medical instruments. In the present text, "transversal" designates a direction which is not collinear with or parallel to a given axis, and is not limited to a perpendicular orientation of the considered direction relative to said given axis. According to an aspect, an angle formed between the main axes of extension of two of the medical instruments is smaller than 15°, advantageously smaller than 10°.

According to a particular embodiment, the main axes of extension of the medical instruments can be parallel to one another. Thus, the medical instruments of the surgical system extend parallel, or sensibly parallel to one another, at least between the robotic arm and the holder. Advantageously, the medical instrument is rigid between the robotic arm and the holder.

The holder comprises at least two distinct pivoting elements, each medical instrument being supported, at least partially, by one of the pivoting elements. Advantageously, the surgical system comprises as many pivoting elements as the number of medical instruments of the surgical system. Each pivoting element can be freely movable around a pivot point and each pivot point can be movable with respect to the other pivot points. Each pivot point can be locked, in position, with respect to the other pivot points. Each pivoting element is movable according to up to five degrees of freedom with respect to the holder.

The robotic assemblies are linked to the holder, by at least one articulated link or by at least one rigid link. The holder is movable with respect to each robotic assembly along at least three translational degrees of freedom. Each medical instrument is attached to one of the robotic arms by a joint configured for the transmission of at least one angular rotation. Optionally, this joint is also configured for the transmission of three additional translations. A first distance measured between the joint formed between the robotic arm and the medical instrument of each robotic assembly and the holder is greater than a second distance measured between the holder and a tip of each medical instrument. This ratio provides a motion scale down between the robotic arm motion and the instrument tip motion. The first distance is at least three times greater than the second distance.

The non-null distance measured between two adjacent medical instruments, in the holder, is comprised between 0,5 mm and 15 mm.

The surgical system according to any of the preceding claims, wherein the robotic assemblies are attached to a base and wherein the base of the surgical system is in a fixed position with respect to a patient support. The patient support can be a chair or a surgical table for instance. Advantageously, the patient can be attached to the chair or to the surgical table, for instance thanks to tape or screws, so that the base of the surgical system is in a fixed position with respect to said patient.

The surgical system comprises a remote-control device configured to displace the robotic arms of the robotic assemblies and to actuate the medical instruments of the robotic assemblies.

Further details and advantages of the invention are described hereunder in relation with the accompanying drawings.
- Figure 1 is a schematic top view of a surgical system;
- Figures 2 and 3 are schematic representations of one robotic assembly of the surgical system according to, respectively, a first and a second embodiment;
- Figure 4 illustrates, schematically, a holder of the surgical system illustrated on figure 1, the holder comprising three pivoting elements and represented in a partially transparent fashion;
- Figure 5 is a focused view of one of the pivoting elements of the holder of the surgical system according to a first example illustrated on figure 4, the holder being only partially illustrated on figure 5;
- Figures 6 and 6a illustrate the holder according to a second example according, respectively, to a frontal view and to a partial cross-section view;
- Figures 7 and 7a illustrate the holder according to a third example according, respectively, to a frontal view and to a partial cross-section view;
- Figure 8 illustrates, in a diagram, two modes of control of robotic assemblies of the surgical system.

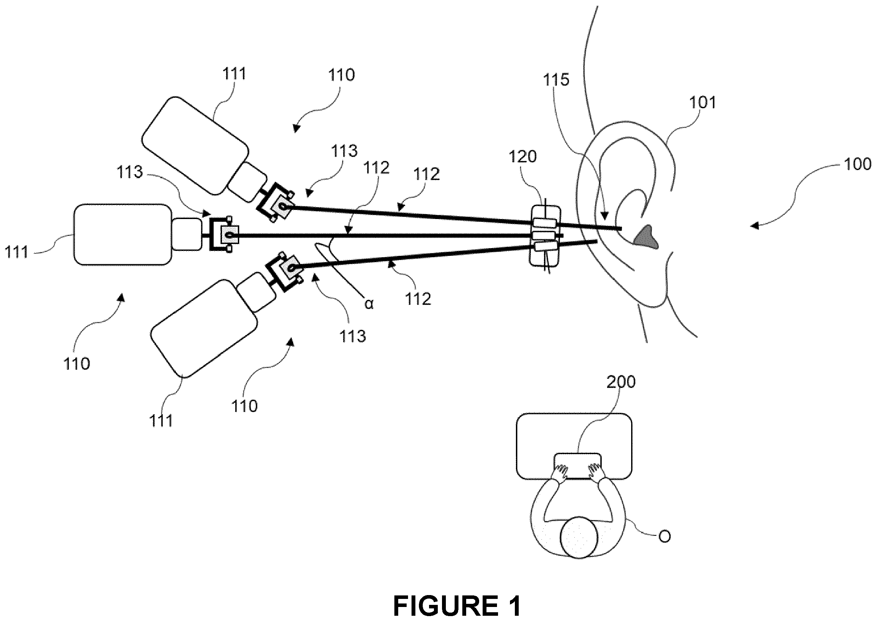

Figure 1 illustrates, schematically, a surgical system 100 according to the invention configured to treat an anatomical structure 101. Figure 1 illustrates the surgical system 100 while used by an operator O for treating an ear, but it could be used for any ENT (Ear, Nose, Throat) surgery within the scope of the invention. As schematically illustrated, the surgical system 100 comprises a remote-control device 200 permitting the operator O to tele-operate said surgical system 100. As detailed below, other modes of control can be implemented within the scope of the invention.

The surgical system 100 comprises at least two robotic assemblies 110. Each robotic assembly 110 comprises at least one robotic arm 111 and one medical instrument 112 held by said robotic arm 111. According to the illustrated example, the surgical system 100 comprises three robotic assemblies 110.

Particularly, each robotic arm 111 extends between a base and a flange, the respective medical instrument 112 being attached to said flange. As detailed below, each medical instrument 112 is attached to one robotic arm by a joint 113 configured for the transmission of one angular rotation and at least one translation from the robotic arm to the medical instrument. According to the particular example illustrated, this joint 113 forms at least part of the flange of the robotic arm 111 and said joint 113 is configured to transmit one angular rotation and three translations. For instance, the joint 113 linking each medical instrument 112 to the respective robotic arm 111 can be a passive joint 113. By "passive joint" we here refer to a joint deprived of motor.

For instance, the joint 113 formed between each robotic arm and the corresponding medical instrument can be a universal joint, a beam joint, a helical joint, diaphragm joint, a flexible or elastomer joint, a jaw or lovejoy joint, a tendon joint, or a boge joint. The joint 113 can be identical for the three robotic assemblies 110 of the surgical system 100 or it can be different for each of them without departing from the scope of the invention.

The surgical system 100 further comprises a holder 120 supporting the three medical instruments 112. This holder 120 is thus common to all the medical instruments of the surgical system 100. This holder 120 is distinct from the flanges of each robotic arm. As detailed below, each medical instrument extends, at least partially, through the holder 120. Advantageously, this holder 120 is made of a sterilizable material.

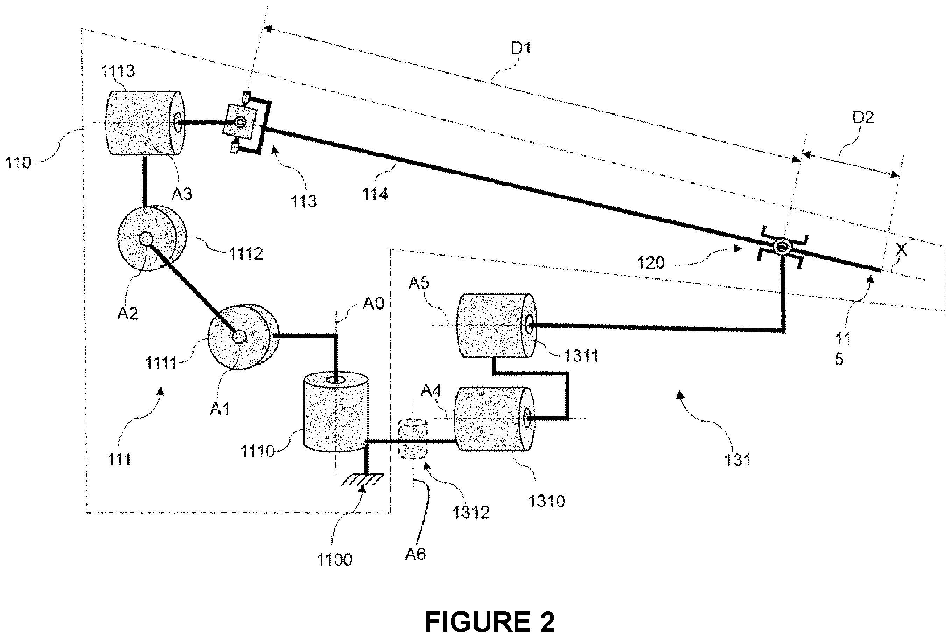

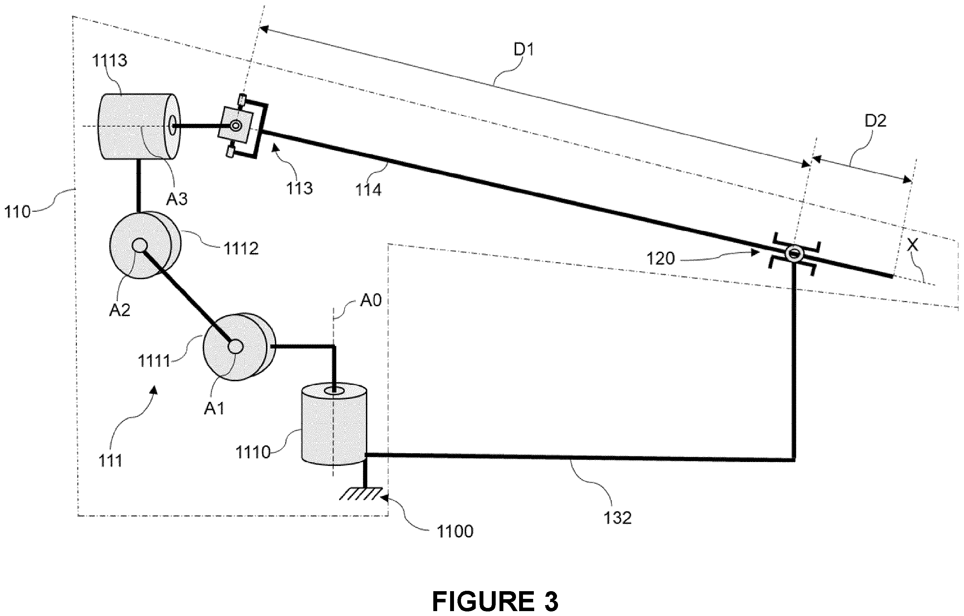

Now referring to figures 2 and 3 we are going to describe one robotic assembly 110 according to, respectively, a first and a second embodiment of the invention. It is understood that the robotic assemblies which are parts of a same surgical system can be identical or not. Particularly, each robotic assembly can comprise a particular number of degrees of freedom, different or not from the other robotic assemblies of the surgical system.

The robotic arm 111 of each robotic assembly 110 presents at least two motorized degrees of freedom. Particularly, the robotic arm 111 is configured to displace the medical instrument 112 according to, at least, one translational degree of freedom and one rotational degree of freedom. As detailed below, the medical instrument 112 extends, at least between the robotic arm 111 and the holder 120, along a main axis of extension X and the robotic arm is configured to displace such medical instrument 112 along this main axis of extension X and around said main axis of extension X. As schematically illustrated on figure 1, an angle α measured between the main axes of extension of two adjacent medical instruments, is smaller than 15°, advantageously smaller than 10°.

According to the illustrated embodiments, each robotic arm 111 comprises at least four motorized degrees of freedom. Especially, a first motorized joint 1110 is a revolute joint around a first axis A0, a second motorized joint 1111 is a revolute joint around a second axis A1, a third motorized joint 1112 is a revolute joint around a third axis A2, and a fourth motorized joint 1113 is a revolute joint around a fourth axis A3. According to the illustrated embodiments, the first axis A0 is perpendicular to the second, the third and the fourth axes A1, A2, A3, the second axis A1 is parallel to the third axis A2 and the fourth axis A3 is perpendicular to the second and the third axis A0, A1, A2.

For instance, the medical instruments 112 are elongated medical instruments, thus comprising an elongated shaft 114 extending along the main axis of extension X, between the joint 113 formed between the robotic arm 111 and the medical instrument 112 and the holder 120 and an effective portion 115 extending beyond the holder 120. Advantageously, the elongated shaft 114 can be formed as a rigid portion. For instance, the effective portion can be adapted to treat the concerned anatomical structure. Alternatively, the medical instrument can be an endoscope, the effective portion thus comprises an imaging apparatus configured to provide images, continuously, of said anatomical structure. Advantageously, one medical instrument of one of the robotic assemblies can be an endoscope while another medical instrument of another robotic assembly can be a surgical instrument, such as a cutting instrument, a forceps or a canula for instance.

According to the illustrated embodiments, the joint 113 formed between the robotic arm 111 and the medical instrument 112 is more particularly formed between the fourth motorized joint 1113 and the medical instrument 112 and said joint 113 is a universal joint. As mentioned above, this joint 113 could be any kind of joint permitting to transmit an angular rotation. Otherwise said, a rotation of the fourth joint 1113 around the fourth axis A3 leads to a rotation of the medical instrument 112 around its main axis X.

According to an aspect of the invention, a first distance D1 measured between the joint 113 formed between the robotic arm 111 and the medical instrument 112 of each robotic assembly 110 and the holder 120 is greater than a second distance D2 measured between the holder 120 and a tip of the medical instrument 112. For instance, the first distance D1 is at least three times greater than the second distance D2. Using such a ratio improves the accuracy when displacing the tip of the medical instrument 112. Advantageously, the first distance D1 is at least four times greater than the second distance D2.

Finally, according to the illustrated embodiments, the surgical system comprises a link 131, 132 linking each robotic assembly 110 to the holder 120, this link being configured to provide at least one transversal degree of freedom to the holder. Particularly, the holder 120 is mobile along, at least, one degree of freedom transversal to the main axes of extension X of the medical instruments.

According to the first embodiment illustrated on figure 2, each robotic assembly 110 is linked to the holder 120 by an articulated link 131 providing motorized degrees of freedom to the holder 120. According to the embodiment shown, this articulated link 131 particularly comprises a first motorized joint 1310 which is a revolute joint configured to rotate around a fifth axis A4, a second motorized joint 1311 which is a revolute joint configured to rotate around a sixth axis A5 parallel to the fifth axis A4. Advantageously, this configuration permits to obtain a holder displaceable according to two motorized degrees of freedom. Optionally, the articulated link 131 could comprise a third motorized joint 1312 arranged before the first and the second motorized joints 1310, 1311 of this articulated link 131 and which would be a revolute joint configured to rotate around a seventh axis A6 secant, advantageously perpendicular, to the fifth and sixth axes A4, A5. As this third motorized joint 1312 is optional, it is illustrated with dotted lines. Obviously, this is only an example and the fifth and sixth axes A4, A5 could be secant within the scope of the present disclosure.

According to the second embodiment illustrated on figure 3, each robotic assembly is linked to the holder 120 by a rigid link 132 configured to provide at least two passive degrees of freedom to the holder 120, i.e., non-motorized degrees of freedom. For instance, the holder 120 could be mounted on a universal passive joint, itself attached to the rigid link 132.

According to a non-illustrated variant of this second embodiment, the rigid link 132 could itself be mounted on a robotic device, for instance on a Selective Compliance Assembly Robot Arm (SCARA), thus providing, at least, three degrees of freedom which can optionally be motorized and configured to be follow tracked movements of the patient, for instance thanks to an optical tracking system, a mechanical tracking system or an electromagnetic tracking system as known by the skilled man of the art. Advantageously, the joints of such a SCARA are lockable.

Obviously, these are only examples of how to carry the invention, but do not limit said invention and other configurations providing robotic arms with at least two, advantageously four, motorized degrees of freedom could be implemented without departing from the scope of the present invention.

Regardless the embodiment implemented, the robotic assemblies 110 can optionally be attached to a base 1100 arranged in a fixed position with respect to a patient support. By "patient support", we here refer to a chair or a surgical table for instance. Advantageously, the patient can be attached to the chair or to the surgical table, for instance thanks to an adhesive element such as tape, or thanks to screws, so that the base 1100 of the surgical system 100 is in a fixed position with respect to said patient, and consequently with respect to the anatomical structure to be treated.

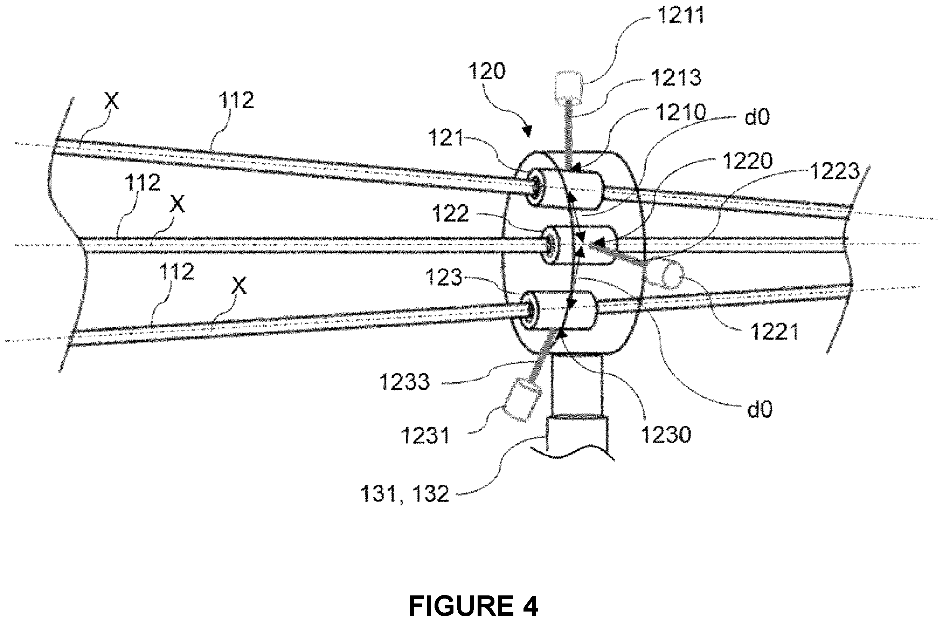

Now referring to figure 4, we are going to describe an example of the holder of the surgical system according to the invention. According to the present disclosure, the holder comprises at least two pivoting elements, each pivoting element supporting, partially, one of the medical instruments.

According to the embodiment shown on figure 4, the holder 120 comprises at least three pivoting elements 121, 122, 123, each supporting, partially, one of the medical instruments 112. According to the particular example illustrated, each pivoting element is here formed as a tubular elemnt, each medical instrument 112 extending, at least partially, through one of the tubular elements 121, 122, 123 of the holder 120. Thus, the pivoting elements 121, 122, 123 also form axial guides to their respective medical instrument. Obviously, this is only an example and the pivoting elements could present any other shape and they could present shapes different from one another without departing from the scope of the invention. According to the invention, each medical instrument 112 is mobile with respect to the holder according to at least two degrees of freedom, namely along and around their respective main axis of extension X. According to the illustrated embodiment, each medical instrument is movable with respect to the holder according to at least four degrees of freedom. Each medical instrument is particularly movable along a longitudinal degree of freedom along its main axis of extension X, around said main axis of extension X, and additionally around a pivot point of the concerned pivoting element as detailed below.

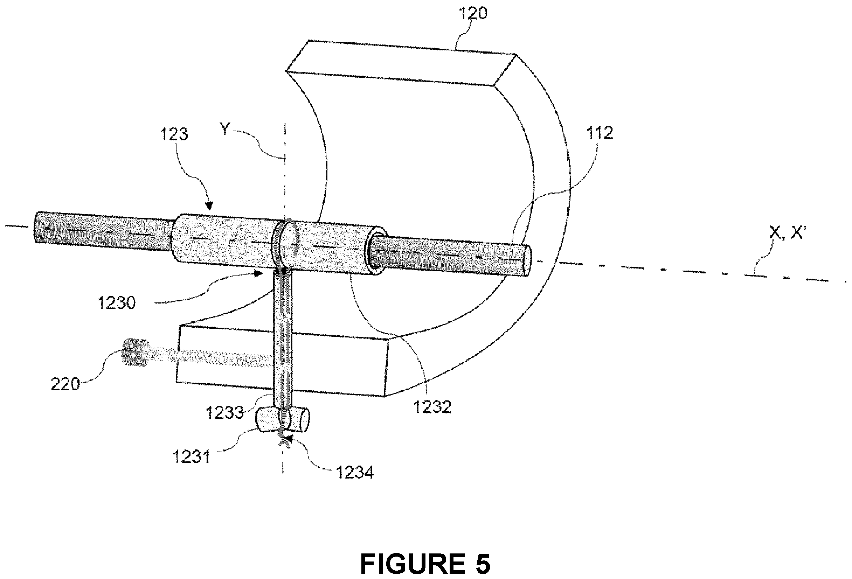

As each medical instrument 112 is driven by its own robotic arm, the medical instruments 112 are mobile with respect to each other and so must be, at least partially, the pivoting elements 121 with respect to one another and with respect to the holder 120. Especially, each pivoting element 121, 122, 123 comprises one fixed pivot point 1210, 1220, 1230 around which it can rotate according to at least two rotational degrees of freedom. Each fixed pivot point 1210, 1220, 1230, of each pivoting element 121, 122, 123 can be fixed in position with respect to the other pivoting elements and with respect to the holder 120. Alternatively, the pivot point of each pivoting element can be movable and lockable with respect to the other pivoting elements and with respect to the holder 120, as for instance described with reference to figure 5.

A non-null distance is formed between two adjacent pivoting elements 121, 122, 123 and consequently, between two adjacent medical instruments 112, this distance being measured at any point of the concerned pivoting elements 121, 122, 123. The non-null distance d0 between two adjacent medical instruments measured within the holder 120, along a line passing through the pivot points 1210, 1220, 1230 of the concerned pivoting elements 121, 122, 123 is thus greater than 0. Advantageously, this non-null distance d0 between two adjacent medical instruments measured within the holder 120 along a line passing through the pivot points 1210, 1220, 1230 of the concerned pivoting elements 121, 122, 123 is comprised between 0,5 mm and 15 mm. According to a particular embodiment, this non-null distance d0 is equal, or sensibly equal, to 2 mm.

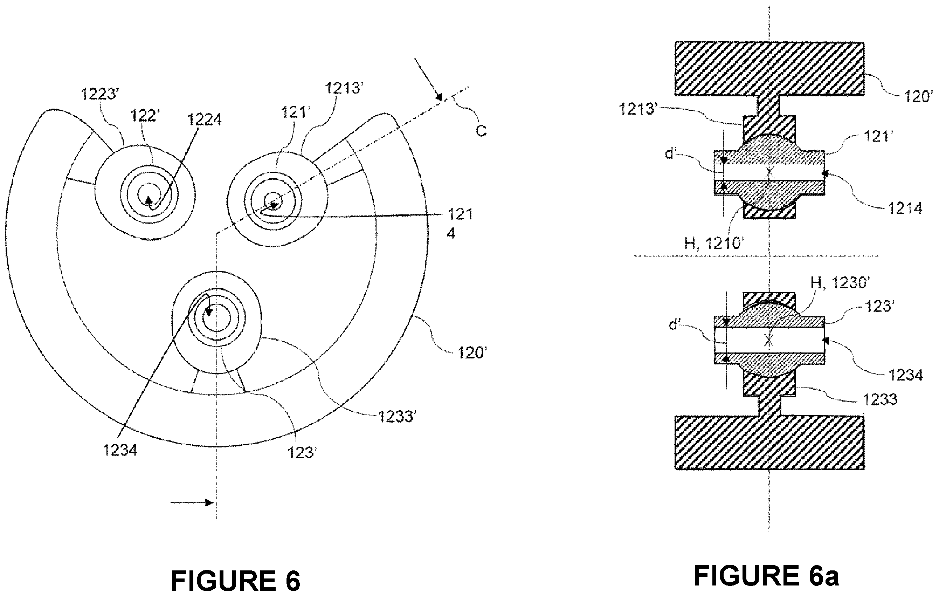

According to a first example illustrated on figure 4, the holder 120 is formed as a hooped element which comprises all three pivoting elements 121, 122, 123. According to the illustrated example, the hooped element is a circular closed hooped element presenting a diameter comprised between 7 mm and 10 mm. Obviously, this is only an example, and the hooped element could present any other shape, such as a square shape, a rectangular shape, an oblong shape or a rhombus shape, opened or closed without departing from the scope of the invention. For instance, figure 6 illustrates an opened holder.

According to the illustrated example, the pivot points 1210, 1220, 1230 are realized by a string attached around each pivoting element 121, 122, 123. Figure 5 is a focused schematic view of one of the pivoting elements 123 illustrated on figure 4.

As illustrated on figure 5, when the pivoting elements are formed as tubular elements, each tubular element 123 presents a T-shape with a first tubular part 1232 through which the medical instrument 112 extends, and a second tubular part 1233 extending, mainly, along a straight-line Y secant, or advantageously perpendicular, to a main axis of extension X' of the first tubular part 1232. The main axis of extension X' of each first tubular part 1232 is advantageously coincident with the main axis of extension X of the medical instrument 112 extending through the concerned tubular element.

According to this example, the pivot point 1230 is realized thanks to a string 1234 which extends and is held within the second tubular part 1233 and circles the medical instrument arranged in the first tubular part 1232. On an opposite side, the string 1234 is attached to an attaching device 1231, also illustrated on figure 4. According to the illustrated embodiment, this attaching device is for instance made of an elastic material, such as rubber or silicone for instance. Alternatively, the attaching device could be realized as a spring.

Alternatively, the string 1234 can be arranged around the first tubular part 1232.

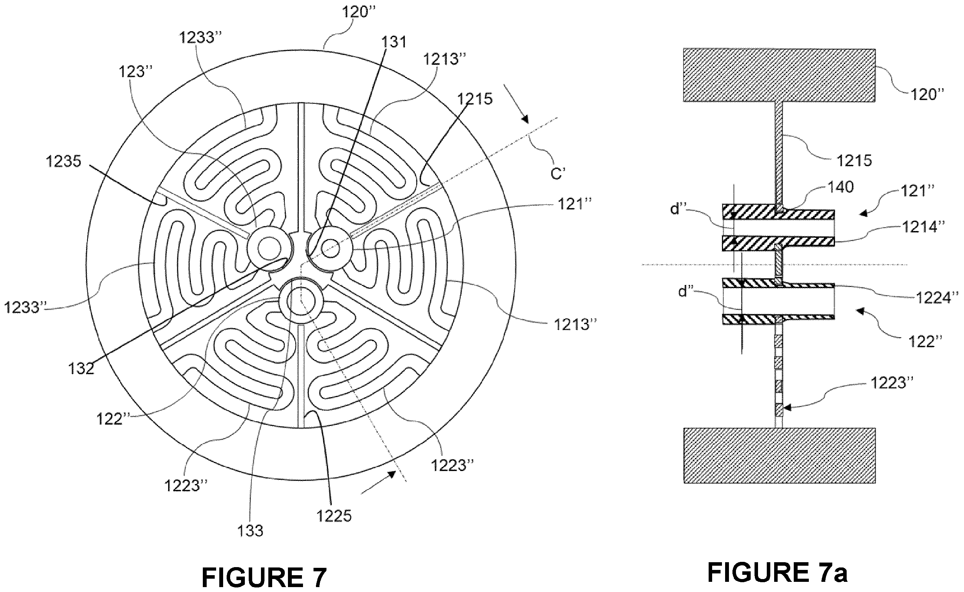

Finally, according to the particular example illustrated, the holder 120 comprises through-holes, here threaded through-holes, through which extend the respective second tubular parts 1233 of each tubular element 123. As schematically represented, those second tubular parts 1233 can be locked thanks to a screw 220. Using a screw advantageously permits to lock and unlock the pivoting element, thus permitting to adjust their respective poses with respect to one another and with respect to the holder 120. Obviously, any other known device could be used. Alternatively, the pivoting elements could be arranged according to fixed positions with respect to the holder, as for instance illustrated on figure 6 and 7.

Figures 6, 6a, 7 and 7a illustrate a second and a third examples of the holder and of the pivoting elements.

Figure 6 and 6a illustrate the second example of the holder 120', according, respectively, to a front view in figure 6, and to a partial cross-section view in figure 6a. Particularly, figure 6a illustrates a cross-section view realized according to the cutting line C illustrated on figure 6.

As illustrated, this second example of the holder 120' differs from the first example in that it presents an opened shape. The pivoting elements 121', 122', 123' according to this second example are also different from the pivoting elements according to the first example in that they are respectively formed as hollowed ball-shaped element configured to receive the elongated shafts of the respective medical instruments. Particularly, each ball-shaped part comprises a bore 1214, 1224, 1234 configured to receive said medical instruments. As illustrated, these bores 1214, 1224, 1234 can present different diameters d', thus being adapted to receive medical instrument of different sizes. Each hollowed ball-shaped part is received in a receptacle 1213', 1223',1233' rigidly attached to the holder 120. Each receptacle presents a complementary shape with respect to the hollowed ball-shaped part which it receives. Particularly, such complementary shapes are configured to permit to its corresponding hollowed ball-shaped part to rotate around its center H. Thus, the center of each hollowed ball-shaped part forms the pivot point 1210', 1230' of the corresponding pivoting element.

As the receptacles are rigidly attached to the holder, the pivot points of the respective pivoting elements are fixed in position with respect to one another and with respect to the holder 120'.

According to the present disclosure, each receptacle can be made of a single piece with the holder, or it can be soldered to the holder.

Figure 7 and 7a illustrate the third example of the holder 120", according, respectively, to a front view in figure 7, and to a partial cross-section view in figure 7a. Particularly, figure 7a illustrates a cross-section view realized according to the cutting line C' illustrated on figure 7.

This third example of the holder 120" differs from the first and second examples in the means implemented to attach the pivoting elements 121", 122", 123" to the holder 120". As illustrated, the pivoting elements 121", 122", 123" are attached to the holder 120" by at least two flexible branches 1213", 1223", 1233" made of elastic material, advantageously elastic metal. By "elastic", we here refer to a material which is adapted to revert to its original shape after a mechanical deformation. Each pivoting element 121", 122", 123" is also partially supported by a rigid branch 1215, 1225, 1235 extending between the two flexible branches which attach the respective pivoting element to the holder, such rigid branch preventing any unwanted displacement of the concerned pivoting element along a direction parallel to said rigid branch 1215, 1225, 1235.

Optionally, the holder 120" according to the third example can comprise a central element comprising three central receptacles 131, 132, 133 respectively configured to receive one of the pivoting elements. Advantageously, these central receptacles present a complementary shape with respect to the shape of an external wall of the pivoting elements.

As illustrated, each pivoting element 121", 122", 123" can be equipped with an adapter 1214", 1224" configured to modify, and particularly reduce, the diameter d" of the concerned pivoting element. Advantageously, these adapters permit to use a unique holder with different kind of medical instruments. As illustrated on figure 7a, each adapter comprises a retainer 140 configured to abut against an external wall of the corresponding pivoting element.

According to a non-illustrated embodiment, the holder could comprise a flexible membrane through which extend the pivoting elements, an interface between the pivoting element and the flexible membrane then forming the pivot points described above.

It is understood that the different examples and embodiment of the pivoting elements and of the holder described above could be combine with one another within the scope of the present disclosure.

As mentioned above, the surgical system can be tele-operated thanks to a remote-control device. According to this example, this remote-control device is configured to control the displacements of the robotic arms and, consequently of the medical instruments held by such robotic arms, and to actuate said medical instruments. This kind of mode of control is well known by the skilled man of the art and is not further detailed in this document.

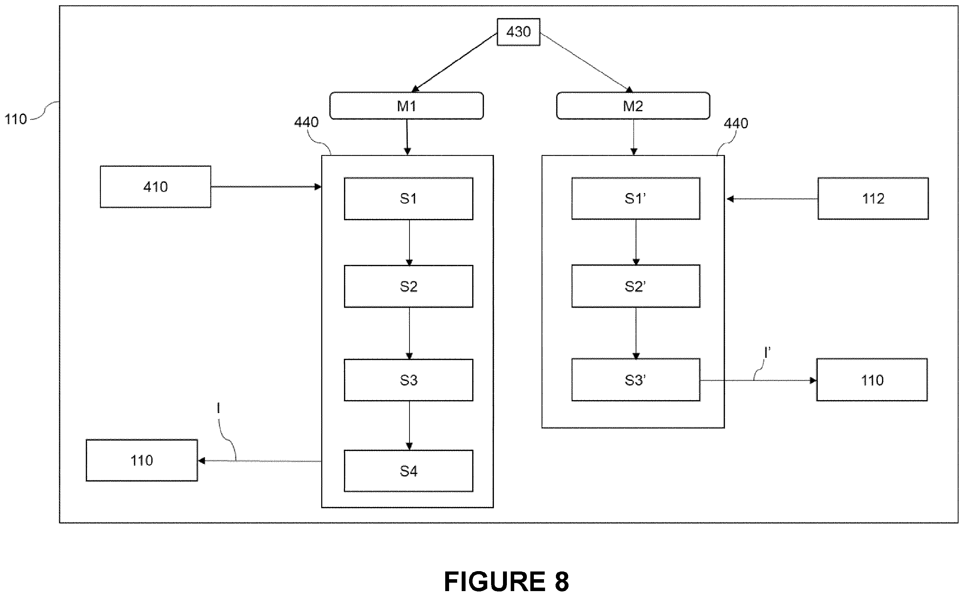

Alternatively, or additionally, at least part of the surgical system, i.e. one or several of the robotic assemblies of this surgical system, can be operated as detailed with reference to figure 8.

Figure 8 illustrates, diagrammatically, two modes of control of the robotic assemblies 110. These modes of control are particularly adapted to control robotic assemblies wherein the medical instruments are endoscopes. Thus, for instance, in a surgical system comprising two robotic assemblies, both modes of control described below with reference to figure 8 can be implemented to control one robotic assembly holding such an endoscope while the other robotic assembly holding a surgical instrument such as a cutting instrument or surgical forceps can be operated thanks to the remote control device.

As schematically illustrated on figure 8, the concerned robotic assembly comprises a trigger mechanism 430 which allows the operator to choose between the two modes. For instance, the trigger mechanism 430 can be formed as a pedal, can be a voice-controlled command, or a button. Any other known kind of trigger mechanism can obviously be implemented within the scope of the present invention.

The concerned robotic assembly 110 can thus be operated according to a head-controlled mode M 1.

As illustrated, the data processor 440 is configured to, as long as the head-controlled mode is enabled:
∘ detect (step s1) at least two successive poses of a face or two successive poses of the eyes of the operator of the system captured by an imaging device 410 configured to capture images of the head of the operator,
∘ determine (step s2) a movement of the face or a movement of the eyes of the operator based on the detected successive poses,
∘ determine (step s3) a commanded displacement of the medical instrument based on the determined movement of the face or of the eyes of the operator,
∘ compute and send (step s4) at least one instruction I to the concerned robotic assembly 110 to displace the medical instrument based on the determined commanded displacement.

The head-controlled mode can also be referred to as a gaze-controlled mode.

Advantageously, the imaging device 410 can be associated with an illumination, such as a LED bar - not illustrated on the figures - to improve the quality of the images captured of the operator by the imaging device. This illumination is particularly configured to ensure that the operator's head is lit, particularly in an operating room where the available lights are mainly directed to the surgical site, while ensuring to not blind the operator.

Optionally, the head-controlled mode can be activated only if commanded by the operator. For instance, the surgical system 100 can comprise an actuator, such as a pedal or a button arranged on the armrest, different from the trigger mechanism, which needs to be actuated to trigger for the imaging device to start capturing images of the head of the operator. Alternatively, some movements can be locked and activated only when the actuator is actuated, while other movements can be unlocked, i.e. continuously used. Particularly, as this mode of control is implemented when the medical instrument is an endoscope, axial translation is often used to advance and pull back the endoscope during the whole duration of the surgery. Then, it would be uselessly complicated to subject the control of such axial translation to any actuator. Rotational movement around its main axis of extension X can also be unlocked as they are safe due the intrinsic shape of an endoscope.

Additionally, the concerned robotic assembly 110 can be operated according to a tool tracking mode M2, the data processor 440 being configured to, as long as the tool-tracking mode is enabled:
∘ identify (step 1') a current pose of a surgical instrument of the other robotic assembly within a field of view of the endoscope 112,
∘ determine (step 2') if the surgical instrument is within a defined zone of the field of view of the endoscope 112,
∘ compute and send (step 3'), when it is determined that the surgical instrument is out of the defined zone, at least one instruction I' to the motorized joints of the robotic assembly 110 to displace the endoscope so as for the surgical instrument to remain within the defined zone of the field of view of the endoscope.
For instance, the defined zone of the endoscope field of view can represent up to 50% of said field of view.

The step of identifying the current pose of the surgical instrument can be realized according to different embodiments. For instance, the surgical tool can be equipped with an optical marker, for instance made as a spot of surgical paint, that the data processor 440 is able to detect. Based on the pose of this optical marker and on a known geometry of the concerned surgical instrument, the data processor can determine the relative pose of such surgical instrument within the field of view of the endoscope.

Alternatively, the data processor can be configured to recognized, in the images captured by the endoscope, the shape of the surgical instrument, based on known technics of image segmentation. Based on this recognition, the data processor is then configured to determine the relative pose of the surgical instrument within the field of view of the endoscope.

Optionally, the surgical system can be configured so that any command of the operator is directly transmitted to the robotic assemblies. Alternatively, the surgical system can be configured to filter and limit the commands of the operator, for instance to implement safety boundaries.

## Claims

1. A surgical system (100) comprising at least two robotic assemblies (110) and a holder (120, 120', 120"), each robotic assembly (110) comprising at least one robotic arm (111) and one medical instrument (112), the robotic arm (111) presenting at least two motorized degrees of freedom and the medical instrument (112) being held by the robotic arm (111), wherein each medical instrument (112) is supported by the holder (120, 120', 120"), and wherein a non-null distance (d0) is formed between two adjacent medical instruments (112), said non-null distance (d0) being measured at any point between the concerned robotic arm (111) and the holder (120, 120', 120").

2. The surgical system (100) according to the preceding claim, comprising at least three robotic assemblies (110).

3. The surgical system (100) according to any of the preceding claims, wherein each robotic arm (111) comprises at least four motorized degrees of freedom.

4. The surgical system (100) according to any of the preceding claims, wherein each medical instrument (112) extends, at least between the robotic arm (111) and the holder (120, 120', 120"), along a respective main axis of extension (X), the holder (120, 120', 120") being movable along at least one translational degree of freedom transversal to the main axis of extension (X) of each medical instrument (112).

5. The surgical system (100) according to any of the preceding claims, wherein the holder (120, 120', 120") comprises at least two distinct pivoting elements (121, 121', 121", 122, 122', 122", 123, 123', 123"), each medical instrument (112) being supported, at least partially, by one of the pivoting elements (121, 121', 121", 122, 122', 122", 123, 123', 123").

6. The surgical system (100) according to the preceding claim, wherein each pivoting element (121, 122, 123) is freely movable around a pivot point (1210, 1220, 1230) and wherein each pivot point (1210, 1220, 1230) is movable with respect to each other pivot point (1210, 1220, 1230).

7. The surgical system (100) according to the preceding claim, wherein each pivot point (1210, 1220, 1230) is lockable, in position, with respect to each other pivot point (1210, 1220, 1230).

8. The surgical system (100) according to any of claims 5 to 7, wherein each pivoting element (121, 121', 121", 122, 122', 122", 123, 123', 123") is movable according to up to five degrees of freedom with respect to the holder (120, 120', 120").

9. The surgical system (100) according to any of the preceding claims, wherein each robotic assembly (110) is linked to the holder (120, 120', 120"), by at least one articulated link (131) or by at least one rigid link (132).

10. The surgical system (100) according to the preceding claim, wherein the holder (120, 120', 120") is movable with respect to each robotic assembly (110) along at least three translational degrees of freedom.

11. The surgical system (100) according to any of the preceding claims, wherein each medical instrument (112) is attached to a respective robotic arm (111) by a joint (113) configured for transmitting at least one angular rotation to the medical instrument.

12. The surgical system (100) according to the preceding claim, wherein a first distance (D1) measured between the joint (113) and the holder (120, 120', 120") is greater than a second distance (D2) measured between the holder (120, 120', 120") and a tip of said medical instrument (112).

13. The surgical system (100) according to the preceding claim, wherein the first distance (D1) is at least three times greater than the second distance (D2).

14. The surgical system (100) according to any of the preceding claims, wherein the non-null distance (d0), measured between two adjacent medical instruments (112), in the holder (120, 120', 120"), is comprised between 0.5 mm and 15 mm.

15. The surgical system (100) according to any of the preceding claims, further comprising a base (1100) configured to be arranged in a fixed position with respect to a patient support, wherein each robotic assembly (110) is attached to the base (1100).

16. The surgical system (100) according to any of the preceding claims, further comprising a remote-control device (200) configured to displace each robotic arm (111) and to actuate each medical instrument (112).
